(19) Europäisches Patentamt
European Patent Office
Office européen des brevets

(11) **EP 4 681 627 A1**

(12) **EUROPEAN PATENT APPLICATION**
published in accordance with Art. 153(4) EPC

(43) Date of publication:
**21.01.2026  Bulletin 2026/04**

(21) Application number: **24795897.8**

(22) Date of filing: **16.04.2024**

(51) International Patent Classification (IPC):
*A61B 5/0537* (2021.01)    *G06F 3/044* (2006.01)

(86) International application number:
**PCT/CN2024/087866**

(87) International publication number:
**WO 2024/222516 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.04.2023  CN 202310483964**

(71) Applicant: **Beijing Tashan Technology Co., Ltd. Beijing 100097 (CN)**

(72) Inventors:
• SUN, Tengchen
  **Beijing 102308 (CN)**
• ZENG, Fanyou
  **Beijing 102308 (CN)**
• WANG, Kai
  **Beijing 102308 (CN)**

(74) Representative: **Yang, Shu**
  **Withers & Rogers LLP**
  **2 London Bridge**
  **London SE1 9RA (GB)**

(54) **SKIN COMPONENT MEASUREMENT MODULE CAPABLE OF ELIMINATING SELF-CAPACITANCE INTERFERENCE OF HUMAN BODY**

(57)    The present invention relates to a skin component detection module capable of eliminating human body self-capacitance interference. It utilizes a structural design to ensure that the proportional coefficient k is known. By measuring the self-capacitance or mutual capacitance of the electrodes twice, the first and second equations between the human body self-capacitance $C_w$ and the actual series capacitance $C_a$ to be measured are established. By solving the system of equations formed by the two equations, $C_w$ can be eliminated, thereby constructing $C_a$ as a monotonic function of the first capacitor and the second capacitor. The $C_a$ is then solved using the first capacitor and the second capacitor obtained from the two measurements. This invention eliminates measurement errors caused by $C_w$ and accurately measures and calculates $C_a$ to reflect the content of skin components.

Figure 1

**Description**

A skin component detection module capable of eliminating human body self-capacitance interference

Technical Field

The present invention relates to the field of skin detection, particularly to a skin component detection module capable of eliminating human self-capacitance interference.

Background

The moisture content in the skin forms a moisturising layer on the skin surface, while oils help the skin retain moisture and inhibit bacteria. The quantitative measurement of both moisture and oil is of significant importance in dermatology and cosmetics.

Traditional methods for detecting moisture or oil in the skin primarily use film measurement techniques, where a film absorbs moisture or oil and then undergoes optical comparison with a standard reference. For example, U.S. Patent No. 4,532,937 discloses a microporous membrane adhered to the skin for absorbing sebum; U.S. Patent 5,119,828 discloses the use of a microporous hydrophobic polymer membrane, which becomes opaque when the pores are filled with gaseous material and semi-transparent when filled with sebum, enabling optical measurement; or German Patent DE29700324U1, which describes skin analysis and assessment using a test membrane, among others. Film measurement methods are indirect measurements that require moisture or oils to transfer to the test film before testing. During transfer, various uncertainties can introduce errors.

To address this, direct measurement methods for testing skin moisture have emerged on the market, such as CK's corneal measurement instrument. Its principle involves using capacitive measurement, where two measurement electrodes form a mutual capacitive electric field that penetrates the skin. The addition of water causes a change in the dielectric constant within the sensing area, and the resulting change in the mutual capacitance value is used to reflect the moisture content in the skin. The main issue with this method is that we aim to measure the series capacitance $C_a$ between the measurement electrode and the skin. However, the skin is an outer layer of the human body, and beneath it lie various conductive substances such as blood. The collective effect of all these substances forms the distributed capacitance (body capacitance) $C_w$. Whether measuring self-capacitance or mutual capacitance, the body's self-capacitance $C_w$ is introduced, leading to inaccuracies in the measurement of the series capacitance $C_a$ and affecting measurement precision. If invasive methods such as cutting a portion of the skin are used to isolate the body's self-capacitance $C_w$, issues such as the loss of activity in the cut skin will also introduce measurement errors. Therefore, the core issue that the direct measurement method in capacitive measurement must address is how to eliminate the adverse effects of $C_w$.

Invention Summary

The objective of this invention is to create a skin component detection module capable of eliminating human body self-capacitance interference, thereby accurately measuring and calculating $C_a$ to reflect skin component content.

The skin component detection module of the present invention includes a capacitance-to-digital conversion circuit (CDC), a processing module, a first measurement electrode, a second measurement electrode, and an insulating layer; The first measurement electrode and the second measurement electrode are used to contact the skin via an insulation layer, and the ratio of the series capacitance Ca1 between the first measurement electrode and the human body to the series capacitance Ca2 between the second measurement electrode and the human body (Ca1/Ca2) is configured to establish a known scaling factor k; the capacitance-to-digital conversion circuit is coupled to the measurement electrodes and obtains the first capacitance and the second capacitance, where the first capacitance is configured to be one of the following: the first self-capacitance measurement value obtained through the first measuring electrode, the second self-capacitance measurement value obtained through the second measuring electrode, the third self-capacitance mea.surement value obtained through the first and second measuring electrodes in parallel, or the first mutual capacitance measurement value obtained through the first and second measuring electrodes; and the second capacitance is configured to be one of the remaining three. Processing module, used to construct a first equation with the human body's distributed capacitance $C_w$ relative to ground and the corresponding series capacitor as variables based on the first capacitor, and a second equation with the human body's distributed capacitance $C_w$ relative to ground and the corresponding series capacitor as variables based on the second capacitor. The series capacitor C is calculated using the system of equations formed by the first and second equations and the proportional coefficient k, $C_{a1}$ or the series capacitance $C_{a2}$ to output the component information within the skin.

In actual scenarios, during skin component detection, the first measurement electrode and the second measurement electrode are tightly pressed against the skin through an insulating layer, with a fixed distance between the electrodes and the skin. By setting the area ratio of the first measurement electrode and the second measurement electrode after production and manufacturing, the proportional coefficient k can be determined through structural design (the series capacitance of one is $C_a$, and the series capacitance of the other is $k*C_a$). Based on this, by measuring the self-capacitance or mutual capacitance between the two electrodes, since both self-capacitance and mutual capacitance are composed of $C_w$ and $C_a$, each measurement can establish a function equation of $C_a$ with $C_w$. By solving the system of equations formed by the two equations, $C_a$ can be determined as a monotonic function of $C_w$, thereby constructing a monotonic function of $C_a$ with the first and second capacitors. a, and by solving the system of equations formed by the two equations, $C_w$ can be eliminated to obtain a monotonic function of $C_a$ with respect to the first and second capacitors. Then, using the first and second capacitors obtained from the two measurements, $C_a$ can be solved. Since $C_w$ is eliminated, the measurement error caused by $C_w$ can be eliminated, thereby accurately measuring and calculating $C_a$ to reflect the skin component content. Additionally, using a capacitive digital converter (CDC), such as the ADI7142 or ADI7147, and employing $\Delta$-$\Sigma$ modulation, the measured capacitance is repeatedly charged and discharged and compared with a reference capacitance. (see: US Patent Number: 5,134,401) to directly convert the measured capacitance value into a digital value. This enhances the measurement sensitivity of capacitance to the 1ff level, easily meeting the measurement system's requirements for capacitance measurement sensitivity. Furthermore, these chips are designed with multiple channels, simplifying circuit design and effectively reducing costs and installation complexity.

As an improved solution, the proportional coefficient k can be configured to be equal to 1 or not equal to 1. When k is equal to 1, this can be achieved by setting the normal projection areas of the first measurement electrode and the second measurement electrode corresponding to the human body to be the same, and the distance between the first measurement electrode and the human body to be the same as the distance between the second measurement electrode and the human body. This scheme is more advantageous for reducing computational power and improving detection speed, and is therefore a preferred solution. For solutions where k is not equal to 1, this can be achieved by setting different areas and/or distances, such as setting the area of the first measurement electrode to half that of the second measurement electrode, or setting the distance between the first measurement electrode and the human body to one-third of the distance between the second measurement electrode and the human body.

As another improved scheme, the first capacitor is configured as the first self-capacitance measurement value or the second self-capacitance measurement value, and the second capacitor is configured as the first mutual capacitance measurement value. In this case, one of the first self-capacitance measurement value and the second self-capacitance measurement value is set as $C_s$, and the corresponding series capacitance is $C_a$; the value of the series capacitance of the other is $k * C_a$. The calculation method of $C_a$ is further configured as:

$$C_a = k_1 * \frac{k*C_s + k*C_x + \sqrt{C_s^2*k^2 - 2C_s*C_x*k^2 + C_x^2*k^2 + 4*C_s*C_x*k}}{2*k} \pm k_2;$$

where $C_x$ is the first mutual capacitance measurement value, $k_1$ is 0.9-1.1, and $k_2$ is the allowable error value. In this scheme, the first capacitor is configured as the self-capacitance, and the second capacitor is configured as the mutual capacitance. The self-capacitance and mutual capacitance conversion method is used to obtain more accurate measurement results.

Alternatively, as an alternative to this improved scheme, the first capacitor can be configured as the first self-capacitance measurement value or the second self-capacitance measurement value, and the second capacitor is configured as the third self-capacitance measurement value. In this case, one of the first self-capacitance measurement values or the second self-capacitance measurement values is set as $C_{s1}$, and the corresponding series capacitance is $C_a$. The value of the series capacitance of the other is $k * C_a$, and the calculation of $C_a$ is further configured as:

$$C_a = k_1 * \frac{C_{s1}*C_{s2}*k}{k*C_{s2} - k*C_{s1} + C_{s2} - C_{s1}} \pm k_2;$$

where $C_{s2}$ is the third self-capacitance measurement value, $k_1$ is 0.9-1.1, and $k_2$ is the allowable error value. In this scheme, the first capacitor is configured as a self-capacitor, and the second capacitor is configured as a self-capacitor after its area is changed (by merging the two electrodes via a analogue switch to make them parallel). The more accurate measurement results are obtained by measuring the self-capacitance twice after changing its area.

As another improved scheme, the distance between the first measurement electrode and the second measurement electrode is set to 0.1mm to 2mm to ensure that both electrodes detect temperature and humidity changes in the same space, thereby avoiding interference caused by significant temperature and humidity differences between the two electrodes due to excessive distance.

In this invention, the component can be moisture or oil. By utilising the characteristic that moisture and oil in the skin are located at different depths (water is deeper than oi), detection at different depths is used to distinguish between water and oil. When measuring oil, the penetration depth of the electric field lines can be controlled to treat moisture and human body capacitance as $C_w$ together. Alternatively, when measuring shallow layers (such as moisture in the epidermal tissue), the penetration depth of the electric field lines can be controlled to treat moisture in deeper layers (such as the dermal tissue and/or subcutaneous tissue) and human body capacitance as $C_w$ together. Alternatively, water-oil separation can be achieved by adjusting the excitation frequency (using frequencies sensitive to water or oil).

Based on this, as another improved scheme, at least three measurement electrodes (including the first and second measurement electrodes) can be configured at different positions. The capacitance-to-digital conversion circuit couples the measurement electrodes through an analogue switch array to selectively combine any two or more measurement electrodes to form a pair of electrodes for detecting mutual capacitance. On the one hand, the measurement electrodes are composed of multiple mutual capacitance groups, which can be switched via an analogue switch array to detect components at different positions, and then take the average value to reduce errors caused by positional differences between measurement times. On the other hand, based on this structure, at least two pairs of electrode groups can be configured by combining them, where the electric field lines of the mutual capacitance formed by each pair of electrode groups have different depths, thereby achieving a change in the penetration depth of the electric field lines. Specifically, variable area and/or variable spacing can be adopted in this structure, i.e., by selecting different configurations, the area or spacing between the two electrodes in each pair of electrode groups can be made different. As a result, the depth of the electric field lines formed by the mutual capacitance of each pair of electrode groups detected by the CDC will vary, thereby enabling detection of different depths or the state of different layers of skin tissue. In the variable area scheme, for example, six electrodes 1-1, 1-2, 1-3, 1-4, 1-5, and 1-6 are set up. The CDC measures the mutual capacitance between 1-2, 1-3, and using a simulated switch array. At this point, the electric field lines of the mutual capacitance penetrate to a shallow depth. Subsequently, by merging (in parallel) 1-1 and 1-2, and 1-3 and 1-4, the mutual capacitance between the two merged electrodes is measured. After merging, the area of the electrodes increases, and the penetration depth of the electric field lines deepens, enabling the testing of skin tissue at different depths at the same location. Alternatively, the mutual capacitance between 1-1 and 1-2 is first measured, then 1-1 and 1-2 are merged, and the mutual capacitance between 1-3 and 1-4 is measured after merging. This allows testing of skin tissue at different depths at the same location. In a variable spacing scheme, the six electrodes can be equally spaced or unevenly spaced. For example, in an evenly spaced configuration, the mutual capacitance between different electrodes is measured first, and then 1 1-4 to measure the mutual capacitance after the area has been changed, thereby testing skin tissue at different depths at different locations. Variable spacing scheme: The aforementioned six electrodes can be evenly spaced or unevenly spaced. For example, in an evenly spaced configuration, 1-1 and 1-2 are used to test the epidermis, 1-1 and 1-4 to test the dermis, and 1-1 and 1-6 to test subcutaneous tissue; or, to simplify wiring, in a different spacing configuration, use 1-1 and 1-2 to test epidermal tissue, 1-3 and 1-4 to test dermal tissue, and 1-5 and 1-6 to test subcutaneous tissue. From the above, it can be seen that, based on the configuration of at least three measurement electrodes distributed at different positions, by combining CDC with a digital switch array, multiple functional objectives can be achieved: (1) Conveniently reduce measurement errors caused by spatial differences between measurement points by averaging; (2) Conveniently achieve variable spacing and/or variable area through combination to detect different depths, including different depths at the same location and/or different locations. In this invention, the analogue switch array can be implemented using an analogue signal router for simple and convenient free combination and switching. For reference, see patent CN202110957486.8, further details are omitted here. Furthermore, the analog signal router can be integrated with a CDC, such as the ruby chip described in CN202110956246.6, to achieve a 24-bit high-speed CDC, an effective resolution of 21.9bits, a conversion time of 0.5ms, and high-precision tactile signal acquisition and encoding.

As a further improvement to the improved scheme, the electrode assembly is configured to include at least two of the following: a first electrode assembly, wherein the spacing and/or area of the two electrodes in the first electrode assembly is configured such that the depth of the electric field lines of the mutual capacitance can penetrate to the epidermal tissue of the skin; a second electrode assembly, wherein the spacing and/or area of the two electrodes in the second electrode assembly is configured such that the depth of the electric field lines of the mutual capacitance can penetrate to the dermal tissue of the skin; a third electrode group, wherein the spacing and/or area of the two electrodes in the third electrode group is configured such that the depth of the electric field lines of the mutual capacitance can penetrate to the subcutaneous tissue of the skin. The epidermis, dermis, and subcutaneous tissue together form the skin. By configuring the electric field lines to penetrate at least two or even all three of these layers, and in conjunction with the CDC and analog switch array, it is possible to achieve a more comprehensive and accurate reflection of skin component parameters. Additionally, this approach allows for selective detection of the state of different tissue layers.

In the aforementioned schemes for detecting different depths or different layers of skin tissue using variable area and/or variable spacing, the fundamental principle is that the frequency of the excitation signal output from the CDC to the measurement electrodes is fixed and unchanged. In another scheme for altering the detection depth, the frequency of the excitation signal can be configured to at least two different frequencies. For example, between two measurement electrodes with fixed spacing and fixed area, such as the first measurement electrode and the second measurement electrode, by configuring the software in the ruby chip, the excitation signal operates at the first frequency during time segment A and at the second frequency during time segment B Due to the different frequencies, under the skin effect, the penetration depth of the mutual capacitance electric field lines between the first measurement electrode and the second measurement electrode also changes, thereby achieving measurements at different depths. It is worth noting that, of course, further frequency changes can be used on the basis of variable area and/or variable spacing to achieve finer depth control. On this basis, it is more preferably configured such that the selected frequencies have different sensitivities to different components in the skin. For example, the first frequency can be configured to be sensitive to water, while the second frequency is sensitive to oils, thereby achieving water-oil differentiation.

As another improved solution, the skin component detection module further includes a first base body and an actuator; the measurement electrodes are arranged on the first base body; and an actuator coupling module is provided to drive the first base body to move, thereby adjusting the contact pressure between the measurement electrodes and the skin. By adjusting the contact pressure between the measurement electrodes and the skin using an actuator (such as a motor), it ensures that the measurement electrodes are tightly pressed against the skin through an insulating layer, thereby avoiding measurement errors caused by loosening or poor adhesion. Furthermore, a pressure sensing component is configured in the coupling processing module. The pressure sensing component detects the contact pressure between the electrodes on the first base and the skin. The processing module adjusts the contact pressure based on the feedback from the pressure sensing component. More preferably, to minimise the introduction of objects between the measurement electrode and the skin that could affect the electrode's measurement, the pressure sensing component uses a capacitive design to indirectly measure the contact pressure (resistive designs are not suitable as they would require a pad between the electrode and the skin). The capacitive pressure sensing component can indirectly reflect pressure through area and/or distance, for example:

For a scheme using distance to reflect pressure, the following configuration can be implemented: the pressure sensing component is configured to include at least a first distance detection electrode and a second distance detection electrode, with the first distance detection electrode fixed to the first substrate on the side opposite the measurement electrode, and the second distance detection electrode configured to be arranged along the direction of movement of the first substrate and aligned with at least part or all of the first distance detection electrode; A capacitive digital conversion circuit is coupled to the first distance detection electrode and the second distance detection electrode to obtain the mutual capacitance between them; A processing module is configured to output the movement distance information of the first base body based on the mutual capacitance between the first distance detection electrode and the second distance detection electrode. Since the first base body is tightly adhered to the skin via an insulating layer, the movement of the first base body is proportional to the contact pressure. Utilising this characteristic, during operation, the movement of the first base body alters the spacing between the first distance detection electrode and the second distance detection electrode, thereby causing a change in their mutual capacitance. The processing module calculates the movement distance of the first base body based on the change in mutual capacitance and converts it into pressure data, achieving the purpose of indirect measurement. At this point, the distance detection electrodes for measuring pressure are located on one side of the first base, while the measuring electrodes are located on the other side, and the two do not interfere with each other. Preferably, to prevent the first distance detection electrodes from being misaligned or tilted relative to the second distance detection electrodes, the pressure sensing assembly is equipped with guide columns. The first base moves in the guided direction with the assistance of the guide columns. The specific structural implementation can be configured such that the first base is mounted on the guide columns.

For schemes where pressure is reflected by area or even a combination of area and distance, a two-dimensional force structure as shown in Patent CN202223551426.5 can be adopted. This involves fixing cylindrical or semi-cylindrical curved surface elastic upper electrodes within a strip-shaped flexible multi-functional layer on the side of the first base body opposite the measuring electrode (with the cylindrical or semi-cylindrical curved surface facing away from the measurement electrode), and at least two lower electrodes distributed on both sides of the upper layer are positioned below the upper layer. The upper and lower electrodes form different capacitances to reflect the components of force in different directions. An insulating layer is placed between the upper and lower electrodes, and the downward projection of the upper electrode covers at least part of the area of each lower electrode. When the first base moves, the upper electrode deforms radially due to the force, causing the upper electrode to change its contact area with the insulating layer, thereby reflecting changes in pressure information. Alternatively, the three-dimensional force structure shown in Patent CN201910370967.1 can be used to achieve higher resolution measurements. In this scheme, indirect measurement is also achieved, and pressure detection is not interfered with by skin detection by the measuring electrodes.

As a further improvement to this modified scheme, the pressure sensing components are configured as at least two units and distributed around each measurement electrode to ensure detection uniformity. Alternatively, the first base is fixed to the skin component detection module via an elastic element (e.g., a spring) to achieve pre-pressure and preliminary control of the contact pressure within a predetermined range.

As another improved solution, the skin component detection module further includes a standard liquid storage device; The standard liquid storage device comprises at least a sealed chamber, a standard liquid corresponding to the component, which is set within the sealed chamber for calibration or differential measurement, and a liquid detection electrode for detecting the capacitance value of the standard liquid under different environmental conditions; a capacitance-to-digital conversion circuit coupled to the liquid detection electrode; and a processing module for correcting the capacitance (self-capacitance, mutual capacitance) obtained by the capacitance-to-digital conversion circuit based on the capacitance value of the standard liquid. When the measured component is moisture, the standard liquid is standard water; when measuring oils, the standard liquid is replaced with an oil. The liquid detection electrodes are configured to include at least two electrodes distributed on the outer wall of the sealed chamber, and the capacitance value of the standard liquid in the chamber is detected via the mutual capacitance between the two electrodes.

In this improved scheme, the method for calibrating the standard liquid further includes detecting the difference between the capacitance value of the standard liquid at the current time and the capacitance value at the initial time, and using the change in the reference reflected by this difference to correct the capacitance obtained by the CDC. For example, assume that the volume of the standard liquid is configured to correspond to full scale. At the initial time, the capacitance value of the standard liquid is detected as A, meaning that the capacitance value A corresponds to full scale. If the capacitance value of the standard liquid detected at the current time is not A but B, this indicates that environmental conditions (such as temperature and humidity) have changed, causing the capacitance to change. At this point, the difference between the capacitance values of the standard liquid from A to B, assuming the same volume, indicates that the baseline has changed. At this point, B corresponds to full scale. Therefore, the capacitance obtained by the CDC must be corrected based on this baseline change.

In this improved scheme, the method of using standard liquid for differential measurement further includes comparing the capacitance value measured by the standard liquid detection electrode with the capacitance value measured by the user through a digital circuit CDC, thereby reducing measurement errors caused by changes in environmental factors (common-mode interference). The standard liquid is stored inside a sealed chamber and does not need to be replaced frequently, reducing the need for users to perform calibration operations during skin testing, achieving real-time calibration and making the operation more convenient.

In the present invention, the insulating layer may be made of flexible or rigid material.

Figure Description

Figure 1 shows a schematic diagram of the skin component detection module structure;

Figure 2-1 shows a partial structural diagram of the detection sensor;

Figure 2-2 shows an equivalent schematic diagram of the distribution capacitance between the detection electrode and the human body relative to ground;

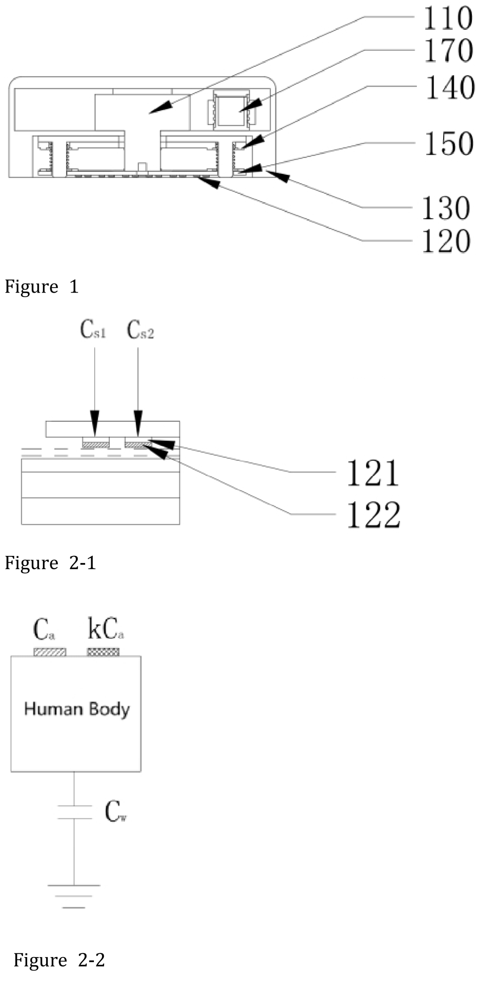

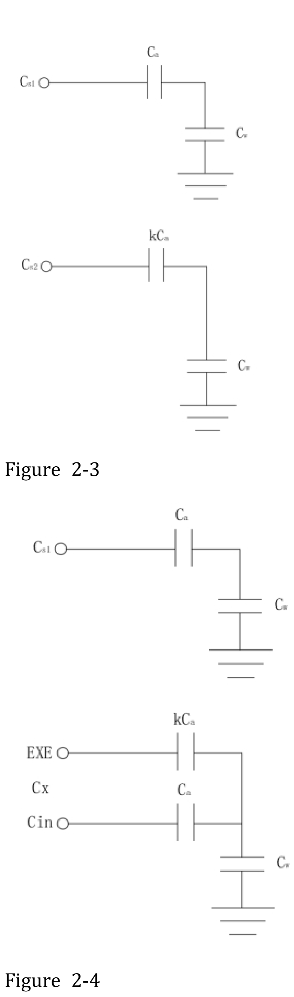

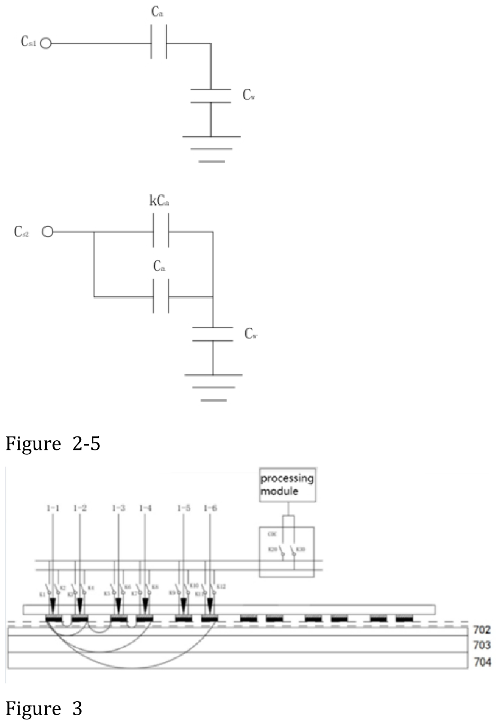

Figure 2-3 shows the capacitance distribution diagram when the first capacitor is configured as the first measurement electrode $C_{s1}$ and the second capacitor is configured as the second measurement electrode $C_{s2}$;

Figure 2-4 shows the capacitance distribution diagram when the first capacitor is configured as the first measurement electrode $C_{s1}$ and the second capacitor is configured as the mutual capacitance between the first detection electrode and the second detection electrode;

Figure 2-5 shows the capacitance distribution diagram when the first capacitor is configured as the first measurement electrode $C_{s1}$ and the self-capacitance measurement value is taken, and the second capacitor is configured as the self-capacitance when the first measurement electrode $C_{s1}$ is connected in parallel with the second measurement electrode $C_{s2}$.

Figure 3 shows a schematic diagram of a switch array for testing different skin layers at various depths using a multi-electrode combination;

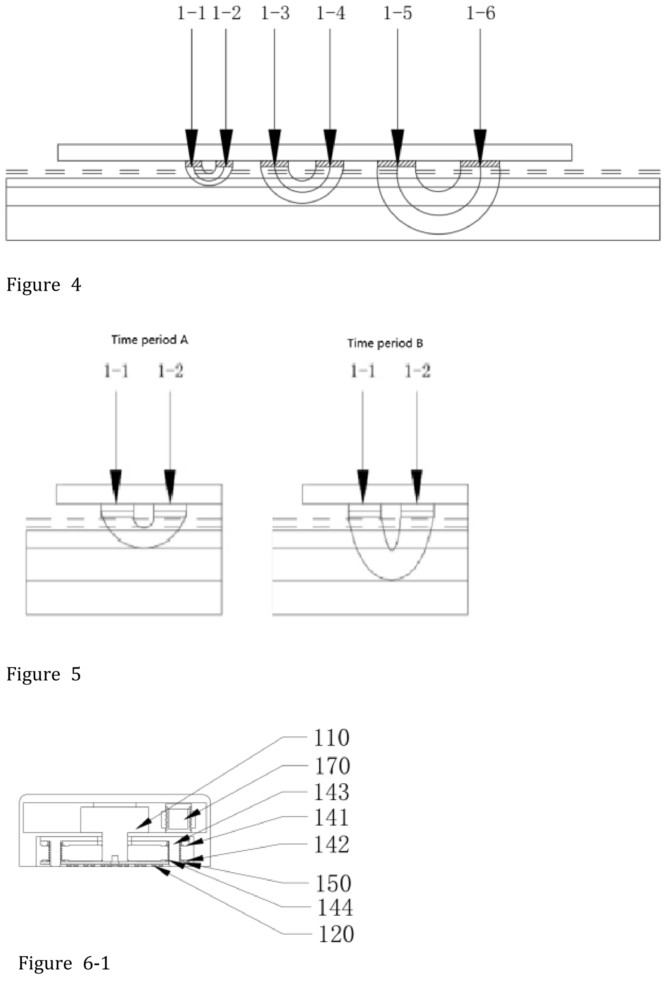

Figure 4 shows a structural schematic diagram of electrode groups with different spacings or areas for testing depth;

Figure 5 shows the structural intent of testing different depths using excitation signals of different frequencies;

Figure 6-1 shows a schematic diagram of the internal pressure detection device structure of the skin component detection module;

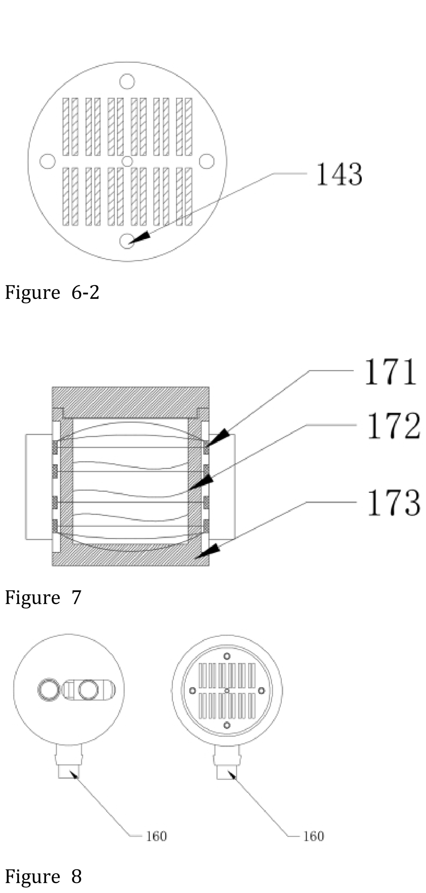

Figure 6-2 shows the structural schematic diagram of the distribution of the first internal pressure detection electrodes and guide columns;

Figure 7 shows a schematic diagram of the standard liquid storage device structure;

Figure 8 shows a schematic diagram of the standard interface structure.

Specific implementation

The technical solutions of the present invention will be described in detail below with reference to the accompanying drawings.

As shown in Figure 1, the skin component detection module includes an internal brake 110, a detection sensor 120, an auxiliary positioning housing 130, an internal pressure detection device 140 (i.e., pressure sensing component), a front-end processing module 150, and a standard liquid storage device 170. The capacitive-to-digital conversion circuit (CDC) is mounted on the front-end processing module 150, such as using an R-SpiNNaker chip. For detailed information, please refer to the patent document CN202110956246.6.

As shown in Figure 2-1, the detection sensor 120 includes detection electrodes 121, each of which has an insulating layer 122. The insulating layer 122 includes, but is not limited to, adhesive insulating tape or an electrode surface coating. The detection electrodes 121 include at least a first measurement electrode $C_{s1}$ (whose series capacitance with the human body is $C_{a1}$) and a second measurement electrode $C_{s2}$ (with a series capacitance of $C_{a2}$ between it and the human body), and the first measurement electrode $C_{s1}$ and the second measurement electrode $C_{s2}$ are configured with a known proportional coefficient k. When the proportional coefficient is set to 1, the first measurement electrode and the second measurement electrode have the same area and the same distance from the human skin. The capacitance digital conversion circuit obtains the first capacitance and the second capacitance, where the first capacitance is configured to be one of the first self-capacitance measurement value obtained through the first measurement electrode, the second self-capacitance measurement value obtained through the second measurement electrode, the third self-capacitance measurement value obtained through the first and second measurement electrodes in parallel, or the first mutual capacitance measurement value obtained through the first and second measurement electrodes, and the second capacitance is configured to be one of the remaining three.

Figure 2-2 shows an equivalent schematic of the distributed capacitance between the detection electrodes and the human body relative to ground. For convenience of conversion and understanding in the following text, for example, the series capacitance between the first measurement electrode $C_{s1}$ and the human body is marked as $C_a$, then the series capacitance between the second measurement electrode $C_{s2}$ and the human body, $C_{a2}$ can be represented as k $C_a$, and the human body's capacitance relative to ground can be equivalently represented as capacitance $C_w$.

$C_a$ Figure 2-3 illustrates the method of using two self-capacitance measurements to calculate the human body's capacitance distribution. In Figure 2-3, the self-capacitance measurement value obtained by the first measurement electrode $C_{s1}$ is used as the first capacitance. The processing module constructs the first equation based on the first capacitance, with the human body's capacitance distribution $C_w$ and the corresponding series capacitance as variables, thereby obtaining the human body's capacitance

$$\text{distribution} C_w = \frac{C_{s1}*C_a}{C_a-C_{s1}} \quad ;$$

$$C_a = \frac{C_{s1}*C_{s2}*k-C_{s1}*C_{s2}}{k*C_{s2}-k*C_{s1}}$$

, The self-capacitance measurement value obtained from the second measurement electrode $C_{s2}$ is used as the second capacitance. Based on the second capacitance, a second equation is constructed with the human body's distributed capacitance $C_w$ and the corresponding series capacitance as variables, yielding the

$$\frac{1}{C_{s2}} = \frac{1}{k*C_a} + \frac{1}{C_w}$$

. By solving the system of equations formed by the first and second equations and using the proportional coefficient k, the human body's distributed capacitance $C_w$ can be determined, and the component information within the skin is output.

Figure 2-4 illustrates the method of measuring $C_a$ using a combination of self-capacitance and mutual capacitance. In Figure 2-4, for example, the self-capacitance measurement value $C_s$ obtained from the first measurement electrode $C_{s1}$ is used as the first capacitance, yielding the equation $\frac{1}{C_s} = \frac{1}{C_a} + \frac{1}{C_w}$. Substituting the known values, we

$$\text{obtain} C_w = \frac{C_s*C_a}{C_a-C_s}$$

. The second capacitance is configured as the second measurement electrode $C_{s1}$ and the second measurement electrode $C_{s2}$ the first mutual capacitance measurement value is $C_x$. The excitation EXE is a square wave with amplitude Ve. Due to the voltage division by kCa and Cw, the excitation voltage across Ca to Cin drops to Ve*k*Ca/(k*Ca+Cw). The essence of capacitor charging and discharging is charge transfer. According to Q=CU, the amount of charge is directly proportional to the voltage. Therefore, the actual measured mutual capacitance value is expressed as $C_x = \frac{k*C_a*C_a}{k*C_a+C_w}$. Substituting $C_w$ into the mutual capacitance equation yields the calculation method for

$C_a$, which is configured $\text{as} C_{a1} = \frac{k*C_s+k*C_x+\sqrt{C_s^2*k^2-2C_s*C_x*k^2+C_x^2*k^2+4*C_s*C_x*k}}{2*k}$. Subsequently, the component information within the skin is output.

Figure 2-5 illustrates the method of combining self-capacitance and variable-area self-capacitance to calculate $C_a$. For example, in Figure 2-5, the self-capacitance measurement value $C_{s1}$ obtained from the first measurement electrode $C_{s1}$ is used as the first capacitance, yielding the equation $C_{s1} = \dfrac{C_a * C_w}{C_a + C_w}$, from which $C_w = \dfrac{C_{s1} * C_a}{C_a - C_{s1}}$ is calculated. The second capacitance is configured as the third self-capacitance measurement value $C_{s2}$, obtained by connecting the first measurement electrode $C_{s1}$ and the second measurement electrode $C_{s2}$ in parallel, and then obtain the self-capacitance through the second measurement electrode $C_{s2}$, yielding the equation $\dfrac{1}{C_{s2}} = \dfrac{1}{C_a + k * C_a} + \dfrac{1}{C_w}$,

solving for $C_{s2} = \dfrac{(C_a + k * C_a) * C_w}{C_a + k * C_a + C_w}$. Thus, the calculation method for $C_a$ is further configured as

$$C_a = \dfrac{C_{s1} * C_{s2} * k}{k * C_{s2} - k * C_{s1} + C_{s2} - C_{s1}}.$$

The above equations $C_a$ are theoretical calculations. In practice, considering measurement errors and allowable tolerances, the calculations for $C_a$ must also incorporate $k_1$, and $k_2$, where $k_1$ ranges from 0.9 to 1.1, and $k_2$ represents the allowable error value, such as $\pm 5\%$ of the measured value.

As shown in Figure 3, the skin component detection module includes at least three sets of electrodes distributed at three different positions. By controlling the switch array through the processing module, different electrode combinations can be tested to measure mutual capacitance values, enabling the detection of skin components at different depths. For example, when detecting epidermal tissue 702, switches K1, K4, K20, and K30 are closed, or K5, K8, K20, and K30 are closed, or K9, K12, K20, and K30 are closed. The electric field line depth can measure the capacitance of epidermal tissue skin components at three different positions, and the average value is calculated to reduce measurement errors caused by positioning errors. When detecting dermal tissue 703, switches K1, K3, K6, and K8 are closed, K20, K30 closed or K5, K7, K10, K12, K20, K30 closed or K2, K4, K5, K7, K20, K30 closed, the depth of the electric field lines can be measured to obtain the capacitance of the dermal tissue skin components at two different positions, and the measurement error caused by the positioning error is obtained by calculating the average. When detecting subcutaneous tissue 704, close switches K1, K3, K10, K12, K20, K30 or K2, K4, K9, K11, K20, and K30 are closed, the depth of the electric field lines can be measured to determine the capacitance of the skin components in the subcutaneous tissue. By increasing the number of electrodes, multiple capacitance values of the skin components in the subcutaneous tissue can be obtained, and the measurement error caused by positioning errors can be reduced by calculating the average value.

As shown in Figure 4, three groups of electrodes with different spacings or areas can be configured. For example, electrode group 1 includes electrodes 1-1 and 1-2 with small surface areas, which can measure epidermal tissue skin components; electrode group 2 includes electrodes 1-3 and 1-4 with moderate surface areas, which can measure dermal tissue skin components; and electrode group 3 includes electrodes 1-5 and 1-6 with large surface areas, which can measure subcutaneous tissue skin components.

As shown in Figure 5, since different components of skin respond differently to excitation signals of different frequencies, at least two excitation frequencies can be configured to detect different components within the same depth range. Additionally, increasing the frequency difference between excitation signals can affect the distribution of electric field lines, thereby controlling the detection depth. During phase A, the excitation frequency is the first excitation frequency, the electric field line depth can measure the capacitance value of the skin component in the epidermal tissue 702. During phase B, the excitation frequency is the second excitation frequency, and the electric field line depth can measure the capacitance value of the skin component in the dermal tissue 703. By combining different electrodes with different frequencies, the testing range can be expanded, and the testing depth can be further refined.

As shown in Figure 6-1, the skin component detection module includes a standard solution storage device 170 and a first base body, The first base body can be a plate, with the detection sensor 120 mounted on the first base body. The first base body is connected to the internal actuator 110 and has a certain axial movement space. The internal actuator 110 can push the first base body to move forward and backward, thereby adjusting the relative position between the detection sensor 120 and the human skin, and consequently regulating the compression degree between the detection sensor 120 and the human skin. The internal pressure sensing assembly comprises an internal pressure first measurement electrode 141, an internal pressure second measurement electrode 142, a guide column 143, and an elastic body 144. The internal pressure first measurement electrode 141 is fixed within the module, and the internal pressure second measurement electrode 142 is fixed on the first base body and aligned with the internal pressure first measurement electrode 141. The first base body makes initial contact with the human skin through the elastic body 144. The mutual capacitance value between the first internal pressure measurement electrode 141 and the second internal pressure measurement electrode 142 can be measured to determine the distance between the first base body and the first internal pressure measurement electrode 141. The pressure value between the first base body and the human skin can be calculated based on the deformation of the elastic body 144 and the holding force of the internal actuator 110. This feedback is transmitted to the front-end processing module 150, which then controls the operation of the internal actuator 110 to adjust the compression degree between the detection sensor 120 and the human skin.

As shown in Figure 6-2, the movement direction of the first base is restricted by at least two guide columns 143 uniformly distributed around the detection centre, ensuring that the movement direction of the first base does not deviate or rotate. Additionally, a set of sensing electrodes is configured at the guide column positions, and a set of first internal pressure measurement electrodes 141 and second internal pressure measurement electrodes 142 are arranged around the guide columns 143. By analysing the capacitance values from different positions, the system can detect whether the skin compression status at different positions remains consistent. Due to the slight axial movement gap between the first base and the internal actuator, the detection sensor 120 initially applies a preload to the human skin through the elastic body 144 when it first contacts the skin, Based on the feedback values from the internal pressure detection electrodes, the deformation of the elastic body is determined, and the internal actuator 110 is controlled to move the first base. When the capacitance value of the internal pressure detection electrodes reaches the preset value, the combined force of the holding force provided by the internal actuator 110 and the deformation force provided by the elastic body 144 reaches the preset value, indicating that the skin compression level detected by the sensor 120 has reached the preset range.

As shown in Figure 7, the standard liquid storage device 170 primarily consists of a standard liquid detection electrode 171, standard liquid 172, and a sealed chamber 173. The standard liquid detection electrode 171 can be a pair of detection electrodes attached to the outer sides of the sealed chamber. In the initial state, it records the initial capacitance value and the corresponding environmental variables. When the environment changes, the capacitance value changes. The change in capacitance of the standard liquid detection electrode 171 can be input into the processing module to perform environmental correction on the test results. Standard solution 172 can be water or standard oil, stored inside the sealed chamber 173. During skin component detection, the digital circuit CDC compares the capacitance values measured by the standard solution detection electrode 171 with those measured by the user, thereby reducing measurement errors caused by changes in environmental factors. Standard solution 172 stored inside the sealed chamber 173 does not require frequent replacement, reducing the need for calibration operations during skin testing and simplifying the process.

As shown in Figure 8, multiple detection modules can be configured for different skin detection projects. These modules can be connected and fixed to skin detection devices, instruments, or wearable skin detection terminals using standard interface 160. The standard interface 160 includes signal output and mechanical fixing clips, enabling interchangeability between detection modules for different skin detection projects.

It should be noted that the above embodiments are provided to illustrate the technical solutions of the present invention and are not intended to limit the scope of protection of the present invention. Although the present invention has been described in detail with reference to the preferred embodiments, those skilled in the art will understand that modifications or equivalent replacements may be made to the technical solutions of the present invention without departing from the essence and scope of the present invention.

**Claims**

1.    , A skin component detection module capable of eliminating human self-capacitance interference, **characterised in that**:

including a capacitance-to-digital conversion circuit (CDC) , a processing module, a first measurement electrode $C_{s1}$, a second measurement electrode $C_{s2}$, and an insulating layer (122) ;
the first measurement electrode $C_{s1}$ and the second measurement electrode $C_{s2}$ are in contact with the skin through the insulating layer (122) and the ratio of the series capacitance Ca1 between the first measurement electrode and the human body to the series capacitance $C_{a2}$ between the second measurement electrode and the human body is configured to be a predetermined known proportional coefficient k;
the capacitance-to-digital(CDC) conversion circuit is coupled to the measurement electrodes $C_{s1}$ and obtains the first capacitance and the second capacitance, wherein the first capacitance is configured to be one of the first self-capacitance measurement value obtained through the first measuring electrode $C_{s1}$ , the second self-capacitance measurement value obtained through the second measuring electrode $C_{s2}$, the third self-capacitance measurement value obtained through the first and second measuring electrodes $C_{s1}$ in parallel, or the first mutual capacitance measurement value obtained through the first and second measuring electrodes $C_{s1}$ , and the second capacitance is configured to be one of the remaining three;
the processing module is configured to construct a first equation based on the first capacitor, where the variables are the human body's distributed capacitance $C_w$ relative to ground and the corresponding series capacitor, and to construct a second equation based on the second capacitor, where the variables are the human body's distributed capacitance $C_w$ and the corresponding series capacitor as variables, and using the system of equations formed by the first equation and the second equation, together with the proportional coefficient k, to calculate the series capacitor $C_{a1}$ or the series capacitor $C_{a2}$ and output the component information within the skin.

2. . According to the skin component detection module claimed in claim 1, the feature lies in that the proportional coefficient k is configured to be equal to 1.

3. . According to claim 2, the skin component detection module is **characterised in that**:

the first measurement electrode $C_{s1}$ and the second measurement electrode $C_{s2}$ correspond to the same normal projection area on the human body;
the distance between the first measurement electrode $C_{s1}$ and the human body is the same as the distance between the second measurement electrode $C_{s2}$ and the human body.

4. . According to the skin component detection module claimed in claim 1, **characterised in that**: the proportional coefficient k is configured to be unequal to 1.

5. . According to the skin component detection module of claim 1, **characterized in that**:

the first capacitor is configured as the first self-capacitance measurement value or the second self-capacitance measurement value, and the second capacitor is configured as the first mutual capacitance measurement value;
where one of the first capacitance measurement value and the second capacitance measurement value is set as $C_s$, and the corresponding series capacitance is set as $C_a$, then the value of the series capacitance of the other is set as k * $C_a$ ;

The calculation method for $C_a$ is further configured as $C_a = k_1 * \dfrac{k*C_s+k*C_x+\sqrt{C_s^2*k^2-2C_s*C_x*k^2+C_x^2*k^2+4*C_s*C_x*k}}{2*k} \pm k_2$ ;

where, $C_x$ is the first mutual capacitance measurement value, $k_1$ is 0.9-1.1, and $k_2$ is the allowable error value.

6. . The skin component detection module according to claim 1, **characterized in that**:

the first capacitor is configured as the first self-capacitance measurement value or the second self-capacitance measurement value, and the second capacitor is configured as the third self-capacitance measurement value;
where one of the first self-capacitance measurement value and the second self-capacitance measurement value is set as $C_{s1}$, and the corresponding series capacitance is $C_a$, then the value of the series capacitance of the other is k * $C_a$ ;

the calculation method for $C_a$ is further configured as $C_a = k_1 * \dfrac{C_{s1}*C_{s2}*k}{k*C_{s2}-k*C_{s1}+C_{s2}-C_{s1}} \pm k_2$ ;

where $C_{s2}$ is the third self-capacitance measurement value, $k_1$ is 0.9-1.1, and $k_2$ is the allowable error value, which is $\pm 5\%$ of the measurement value.

7. . The skin component detection module according to claim 1, **characterized in that** the distance between the first measurement electrode $C_{s2}$ and the second measurement electrode $C_{s2}$ is configured to be 0.1mm to 2 mm.

8. . According to the skin component detection module of claim 1, the feature is that:

the skin component detection module is provided with at least three measurement electrodes distributed at different positions;
the capacitance-to-digital (CDC) conversion circuit is coupled to each measurement electrode via an analog switch array to selectively combine any two or more measurement electrodes to form a pair of electrodes for detecting mutual capacitance.

9. . According to the skin component detection module claimed in claim 8, the feature is that:
at least two pairs of the electrode groups are provided, wherein the electric field lines of the mutual capacitance formed by each pair of electrode groups have different depths.

10. . According to the skin component detection module of claim 9, the feature is that the electrode group is configured to include at least one of the following:

a first electrode group, wherein the spacing and/or area between the two electrodes in the first electrode group is configured such that the electric field lines of the mutual capacitance can penetrate to the epidermal tissue of the skin (702);
a second electrode group, wherein the spacing and/or area between the two electrodes in the second electrode group is configured such that the depth of the electric field lines of the mutual capacitance can penetrate to the dermis of the skin (703);
a third electrode group, wherein the spacing and/or area between the two electrodes in the third electrode group is configured such that the depth of the electric field lines of mutual capacitance can penetrate to the subcutaneous tissue of the skin.

11. . The skin component detection module according to claims 8, 9, or 10, **characterized in that**:
the excitation signal output from the capacitance digital conversion (CDC) circuit to the electrode group is configured to include at least two different frequencies.

12. . According to claim 11, the skin component detection module is **characterised in that**: each frequency has a different sensitivity to different components in the skin.

13. . According to claim 1, the skin component detection module is **characterised in that**:

the skin component detection module further comprises a first base body and an actuator;
the measurement electrodes are arranged on the first substrate;
the actuator is coupled to the processing module to drive the first substrate to move, thereby adjusting the contact pressure between the measurement electrodes and the skin.

14. . The skin component detection module according to claim 13, **characterized in that**:
the skin component detection module further comprises a pressure sensing component coupled to the processing module, wherein the pressure sensing component is configured to detect the contact pressure between the electrodes on the first substrate and the skin.

15. . According to claim 14, the skin component detection module is **characterised in that**:
the pressure sensing component is configured as a capacitive pressure sensing component.

16. . According to claim 15, the skin component detection module is **characterised in that**:
the pressure sensing component is configured as at least two and distributed around each measurement electrode.

17. . According to claim 14, 15, or 16, the skin component detection module is **characterised in that**:
the pressure sensing component further comprises a guide column (143), wherein the first base body is guided in its movement direction by the guide column.

18. . According to claim 13, the skin component detection module is **characterised in that**:
the first base body is fixed (144) to the skin component detection module via an elastic body to achieve pre-compression.

19. . According to the skin component detection module of claim 1, **characterized in that** (170):

the skin component detection module further comprises a standard173 liquid storage device;
the standard liquid storage device comprises at least a sealed chamber173, a standard liquid corresponding to the component for calibration or differential measurement disposed within the sealed chamber, and a liquid172 detection electrode172 for detecting the capacitance value of the standard liquid under different environmental conditions;
the capacitance-to-digital (CDC) conversion circuit is coupled to the liquid detection electrodes;
the processing module is configured to correct the capacitance obtained by the capacitance-to-digital (CDC) conversion circuit based on the capacitance values of the standard liquids.

Figure 1

Figure 2-1

Figure 2-2

Figure 2-3

Figure 2-4

Figure 2-5

Figure 3

Figure 4

Figure 5

Figure 6-1

Figure 6-2

143

171

172

173

Figure 7

160

160

Figure 8

## INTERNATIONAL SEARCH REPORT

| International application No. |
| --- |
| **PCT/CN2024/087866** |

| A. CLASSIFICATION OF SUBJECT MATTER |
| --- |
| A61B 5/0537(2021.01)i; G06F3/044(2006.01)i |

According to International Patent Classification (IPC) or to both national classification and IPC

| B. FIELDS SEARCHED |
| --- |

| Minimum documentation searched (classification system followed by classification symbols) |
| --- |
| IPC: A61B 5/-; G06F 3/- |

Documentation searched other than minimum documentation to the extent that such documents are included in the fields searched

| Electronic data base consulted during the international search (name of data base and, where practicable, search terms used) |
| --- |
| CNABS, CNTXT, DWPI, WPABSC, ENTXTC: 北京他山科技有限公司, 电极, 极板, 水, 油, 组分, 人体, 身体, 组织, 自电容, 绝对电容, 互电容, 跨电容, 数字转换, 干扰, 噪声, 方程, 消元, electrode, capacity, capacitance, CDC, body, human, self, mutual, disturb+, interference, skin |

| C. DOCUMENTS CONSIDERED TO BE RELEVANT |
| --- |

| Category* | Citation of document, with indication, where appropriate, of the relevant passages | Relevant to claim No. |
| --- | --- | --- |
| PX | CN 117017260 A (BEIJING TASHAN TECHNOLOGY CO., LTD.) 10 November 2023 (2023-11-10) <br> claims 1-19 | 1-19 |
| A | CN 115705110 A (SAMSUNG DISPLAY CO., LTD.) 17 February 2023 (2023-02-17) <br> description, paragraphs 128-160 | 1-19 |
| A | CN 107582215 A (BEIJING ZHONGSHUO ZHONGLIAN INTELLIGENT ELECTRONIC TECHNOLOGY CO., LTD.) 16 January 2018 (2018-01-16) <br> entire document | 1-19 |
| A | US 2014049505 A1 (RADIVOJEVIC, Zoran et al.) 20 February 2014 (2014-02-20) <br> entire document | 1-19 |
| A | CN 107174245 A (ANHUI JINJIANQIAO MEDICAL TECHNOLOGY CO., LTD.) 19 September 2017 (2017-09-19) <br> entire document | 1-19 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **14 June 2024** | **19 June 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/087866** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 109381164 A (BEIJING XIAOMI MOBILE SOFTWARE CO., LTD.) 26 February 2019 (2019-02-26)<br>entire document | 1-19 |
| A | US 2015051468 A1 (TEXAS INSTRUMENTS INC.) 19 February 2015 (2015-02-19)<br>entire document | 1-19 |

Form PCT/ISA/210 (second sheet) (July 2022)

**INTERNATIONAL SEARCH REPORT**
Information on patent family members

International application No.

**PCT/CN2024/087866**

| Patent document cited in search report | | | Publication date (day/month/year) | Patent family member(s) | | | Publication date (day/month/year) |
|---|---|---|---|---|---|---|---|
| CN | 117017260 | A | 10 November 2023 | None | | | |
| CN | 115705110 | A | 17 February 2023 | KR | 20230022311 | A | 15 February 2023 |
| | | | | US | 2023042134 | A1 | 09 February 2023 |
| CN | 107582215 | A | 16 January 2018 | None | | | |
| US | 2014049505 | A1 | 20 February 2014 | US | 9110545 | B2 | 18 August 2015 |
| CN | 107174245 | A | 19 September 2017 | None | | | |
| CN | 109381164 | A | 26 February 2019 | None | | | |
| US | 2015051468 | A1 | 19 February 2015 | US | 11596320 | B2 | 07 March 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4532937 A
- US 5119828 A
- DE 29700324 U1
- US 5134401 A
- CN 202110957486
- CN 202110956246
- CN 202223551426
- CN 201910370967